# EUROPEAN PATENT SPECIFICATION

(11) **EP 2 377 465 B1**
(45) Date of publication and mention of the grant of the patent: **27.08.2014**
(21) Application number: 11162484.7
(22) Date of filing: 14.04.2011
(51) Int. Cl.: A61B 5/145, G01N 33/487, G02B 6/00

(54) **Biological information measurement device, illumination method in a biological information measurement device, and biological information measurement method**
Vorrichtung zur Messung biologischer Informationen, Beleuchtungsverfahren in einer Vorrichtung zur Messung biologischer Informationen und Verfahren zur Messung biologischer Informationen
Dispositif de mesure d'informations biologiques, procédé d'éclairage dans un dispositif de mesure d'informations biologiques et procédé de mesure d'informations biologiques

(30) Priority: 14.04.2010 JP 2010093571; 16.03.2011 JP 2011057911
(43) Date of publication of application: 19.10.2011
(73) Proprietor: Arkray, Inc., Minami-ku Kyoto-shi Kyoto 601-8045 (JP)
(72) Inventor: Nishiyama, Hisashi, Kyoto-shi Kyoto 602-0008 (JP)
(74) Representative: MacDougall, Alan John Shaw

(56) References cited:
- EP-A1- 2 103 251
- WO-A2-01/08551

## Description

### BACKGROUND OF THE INVENTION

### Field of the invention

The present invention relates to a measurement device that measures biological information of a user, to an illumination method in a biological information measurement device, and to a biological information measurement method.

### Description of the Related Art

The operability of measurement devices for measuring blood glucose levels, as well as the preparations required for the measurements, among other considerations, are significant issues to users such as diabetic patients who have to measure blood glucose levels and who must control food intake amounts and exercise amounts, as instances of health management. Therefore, portable measurement devices have been developed in order for users to be able to measure blood glucose levels comparatively easily in the daily life. These devices incorporate various functions that help the user manage blood glucose levels.

Ordinarily, sensors in portable measurement devices are disposable: upon each measurement, a new sensor is fitted to the sensor, and blood to be measured is placed on the sensor. In order to facilitate measurements in comparatively dark rooms, for instance during nighttime, measurement devices, which incorporate an illumination device that illuminates the vicinity of a region at which the blood is placed, have been developed (for instance, Japanese Patent Application Publication No. 2006-284481). In the measurement device disclosed in Japanese Patent Application Publication No. 2006-284481, the fingers of the user that performs the blood sampling are illuminated in a spotlight fashion using a light-emitting diode or the like.

WO 01/08551 A2 discloses a glucose monitoring device suitable for use in total darkness or in limited light environment. The glucose monitoring device comprises a housing, which encloses the components of the device that determine the blood glucose level of a blood sample on a test strip. The exterior surface of the housing comprises a phosphorescent material. The portion of the test strip where the blood sample is to be applied can be illuminated by a light. The area of the glucose monitoring device where the test strip is inserted into the device can also be illuminated by a light. The display of the device, i.e., the area of the glucose monitoring device where the result is read, is also illuminated by a light.

EP 2103251 A1 discloses a method of operating an analyte measurement device. The device has a display, a user interface, a processor, a memory and a number of user interface buttons. An analyte is measured with the analyte measurement device and a value representative of the analyte is displayed. The user is prompted to activate a test reminder and the test reminder is activated to remind the user to conduct a test measurement at a different time. To activate a lest reminder, the user presses one of the user interface buttons to turn the analyte measurement device on, and in response to a prompt to confirm the selection of a test reminder, presses one of the user interface buttons to confirm selection of the test reminder. The prompting may comprise repetitively flashing on the display an icon representative of one of the user interface buttons, or illuminating one of the user interface buttons, to prompt a selection of such user interface button.

When considering a situation where devices for measuring biological information such as blood glucose levels are used, it is found that such devices are used not only during daytime, but are also often used in comparatively dark surroundings, for instance during nighttime. Therefore, user-friendliness upon fitting/removal of the sensor for biological information measurement to/from the device main body is directly linked to operability of the measurement device itself. Meanwhile, especially for portable measurement devices, limiting to the utmost the power consumption in the portable measurement device itself is a further requirement, in response to growing environmental awareness.

Thus, user-friendliness has been enhanced in conventional art through arrangement, in the measurement device, of an illumination device (light-emitting diode or the like) for illumination of a blood-sampling site. However, the illumination device entails conversely greater power consumption and a shorter life of the power supply of the portable measurement device.

### SUMMARY OF THE INVENTION

In the light of the above problems, it is an object of embodiments of the invention to provide, in a measurement device that measures biological information of a user, an illumination function that improves user-friendliness, while limiting as much as possible increases in power consumption in the measurement device.

The present invention is a biological information measurement device as defined in Claim 1 of the appended claims. Also provided is an illumination method as defined in Claim 8.

According to one aspect, the present invention is a biological information measurement device in which light emitted by a light source section that already functions as a light source for any component of the device, is guided, by way of a light guide member, to a predetermined site to/from which a sensor for measurements is fitted/removed. As a result, it becomes unnecessary to provide a new light source for sensor fitting/removal, and thus wasteful increases in power consumption in the measurement device are avoided.

Specifically, embodiments provide a biological information measurement device that measures biological information of a user, including a device main body; a fitting-removal section which is provided in the device main body, and to/from which a sensor that measures the biological information is fitted/removed; a light source section that is provided within the device main body at a position spaced apart from the fitting-removal section and that functions as a light source for components of the biological information measurement device other than the fitting-removal section; and an elongate light guide member having a first end portion in a vicinity of the light source section and a second end portion in a vicinity of the fitting-removal section, the elongate light guide member extending from the first end portion to the second end portion for guiding light emitted by the light source section to a predetermined position in the vicinity of the fitting-removal section to/from which the sensor is fitted/removed, wherein the elongate light guide member is arranged such that, in use, light emitted by the light source section is incident into the first end portion, is guided along the elongate light guide member, and exits through the second end portion.

In the biological information measurement device according to this aspect, for instance, the sensor for measurements is fitted to the fitting-removal section of the device main body. In that state, a sample to be measured is, for instance, placed on or absorbed by the sensor, as a result of which a signal relating to the biological information to be measured is transmitted by the sensor to the device main body. Biological information ultimately required by the device main body is generated on the basis of that signal. To measure the desired biological information using a biological information measurement device having such a configuration, the user must fit the sensor to the fitting-removal section, and remove the sensor once the measurement is over. Therefore, the operations of fitting and removing the sensor to/from the device main body upon operation of the measurement device by the user are found to be factors that significantly influence both the user-friendliness and the operability of the measurement device.

In the biological information measurement device, therefore, the light source section functions as a light source for components of the biological information measurement device other than the fitting-removal section, at a position spaced apart from the fitting-removal section. The light source section is built-in as a component already present in the biological information measurement device, and not a new component that is built into the device to solve the above-described problems. In the biological information measurement device, therefore, an already built-in component that functions as any light source corresponds to the above-described light source section.

However, by arranging the light guide member between the light source section and a predetermined position in the vicinity of the fitting-removal section, it becomes now possible to guide light from a light source section, not intended originally for illumination of the fitting-removal section, to the vicinity of the fitting-removal section, and to assist thereby fitting/removal of the sensor to/from the fitting-removal section. In particular, the light that is guided by the light guide member assists effectively the operation sensor fitting/removal operation by the user in a dimly-lit environment, so that light emission that accompanies such assistance does not consume power directly. Therefore, no unnecessary increase in power consumption is incurred by the biological information measurement device. The predetermined position may be any position from which light transmitted by the light guide member can be emitted and at which the sensor Litting/removal operations can be assisted. Such a predetermined position is for instance a position, suitable for the above-described assistance, in the vicinity of the fitting-removal section. The predetermined position may be the position of the fitting-removal section itself.

The biological information measurement device may further include a display that displays biological information measured by way of the sensor. In this case, the light source section may be a backlight that functions as a light source for the display. The backlight is a light source for making it easier for the user to view the contents displayed on the display; i.e. the backlight is a component already built into the measurement device. In a biological information measurement device having such a configuration, therefore, light emitted by the backlight is guided to the predetermined position by the light guide member. As a result, illumination of the predetermined position can be secured without providing any new light source.

In such a case, the fitting-removal section may be provided on a side face of the device main body; and the light guide member may have a length enabling light of the backlight to be guided to the predetermined position. That is, setting an appropriate length for the light guide member allows reliably conveying the light of the backlight to a distant position at which the fitting-removal section is provided.

The abovementioned biological information measurement device may further include an operation unit for enabling a user to operate the biological information measurement device. In this case, the biological information measurement device may be configured so that, upon operation of the operation unit by the user, the light source section emits light and the light guide member guides the light to the predetermined position. In a case where the user operates the biological information measurement device via the operation unit, specifically, fitting of the sensor to the fitting-removal section and subsequent removal of the sensor therefrom can be made easier thanks to light emitted by a light-emitting section, upon contact with the operation unit.

The biological information measurement device may be configured so that upon fitting of the sensor to the fitting-removal section, the light source section emits light and the light guide member guides the light to the predetermined position. In such a configuration, light is guided to a predetermined position, by way of a light guide member, after fitting of the sensor. Therefore, the user can perform more easily various manipulations on the fitted sensor, for instance placement of a biological sample. In that case, light may be emitted by the light source section simultaneously with fitting of the sensor to the fitting-removal section, or after fitting of the sensor.

In the above-mentioned biological information measurement device, the biological information measurement device may be a device that measures a blood glucose level of a user, in which case a sensor for blood glucose level measurement, on which blood of the user is placed and which produces a current output according to a glucose level of the blood, may be fitted to/removed from the fitting-removal section. The biological information measurement device may be a user-portable device. The foregoing features are examples, and the biological information measurement device according to the present invention is not limited to those features.

Another aspect of the present invention is an illumination method in a biological information measurement device. Specifically, the present invention also provides an illumination method in a biological information measurement device that measures biological information of a user and that has a device main body and a fitting-removal section to/from which a sensor that measures biological information of a user is fitted/removed from outside, the illumination method being a method for illuminating a vicinity of the fitting-removal section and including: a first step of causing light emission by a light source section that is provided within the device main body at a position spaced apart from the fitting-removal section and that functions as a light source for components of the biological information measurement device other than the fitting-removal section; and a second step of guiding light emitted by the light source section in the first step, to a predetermined position in the vicinity of the fitting-removal section to/from which the sensor is fitted/removed, by way of an elongate light guide member having a first end portion in a vicinity of the light source section and a second end portion in the vicinity of the fitting-removal section, the elongate light guide member extending from the first end portion to the second end portion, wherein the light emitted by the light source section is incident into the first end portion, is guided along the elongate light guide member, and exits through the second end portion. As is the case of the above-described biological information measurement device, in the illumination method according to the present invention as well illumination is provided during fitting/removal of the sensor, without providing any new light source for sensor fitting/removal. Therefore, device operability and user-friendliness can be enhanced without unnecessary rises in power consumption in the biological information measurement device. The technical ideas disclosed for the biological information measurement device apply in the same way to the illumination method according to the present invention.

The measurement device that measures biological information of a user provides thus an illumination function that improves user friendliness and that allows limiting, as much as possible, increases in power consumption in the measurement device.

### BRIEF DESCRIPTION OF THE DRAWINGS

Fig. 1A is a first diagram illustrating the outer configuration of a blood glucose level measurement device according to the present invention;
Fig. 1B is a second diagram illustrating the outer configuration of a blood glucose level measurement device according to the present invention;
Fig. 2 is a functional block diagram of each functional unit formed in the blood glucose level measurement device illustrated in Fig. 1;
Fig. 3 is a diagram illustrating a display example of a liquid crystal screen in the blood glucose level measurement device illustrated in Fig. 1;
Fig. 4 is an enlarged diagram of the vicinity of a test piece insertion groove in the blood glucose level measurement device illustrated in Fig. 1;
Fig. 5 is a top-view diagram of the blood glucose level measurement device illustrated in Fig. 1, with a front panel removed;
Fig. 6 is a cross-sectional diagram, along A-A, of the blood glucose level measurement device illustrated in Fig. 5;
Fig. 7 is diagram illustrating schematically a horizontal cross section of a blood glucose level measurement device according to a variation; and
Fig. 8 is diagram illustrating schematically a vertical cross section of a blood glucose level measurement device according to a variation.

### DESCRIPTION OF THE PREFERRED EMBODIMENTS

An explanation follows next, with reference to accompanying drawings, on a blood glucose level measurement device that corresponds to the biological information measurement device according to an embodiment of the present invention. The features in the embodiments described below are illustrative in nature, and the present invention is not limited to the features of the embodiments.

### [Embodiment 1]

### (Overview of the blood glucose level measurement device)

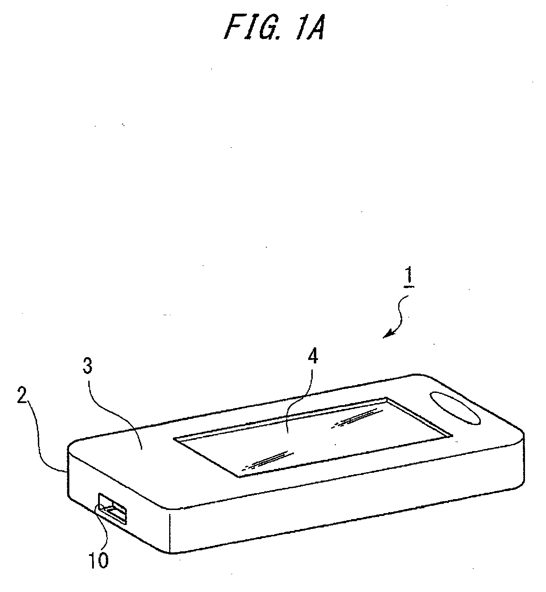

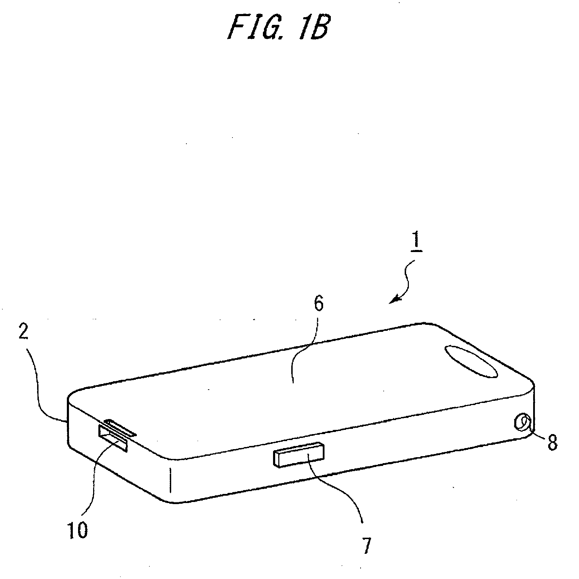

Fig. 1 is a diagram illustrating the outer appearance of a blood glucose level measurement device 1 according to the present invention. The blood glucose level measurement device 1 is a portable measurement device configured in such a manner that a diabetic user can measure blood glucose on his/her own. As illustrated in the figure, the blood glucose level measurement device 1 has a long and thin chassis 2 (device main body), about the size of the user's palm. As a result, operability by the user is enhanced, and measurement results can be managed and displayed, quickly and appropriately, as described below. The device contributes thus to the blood glucose management of the user.

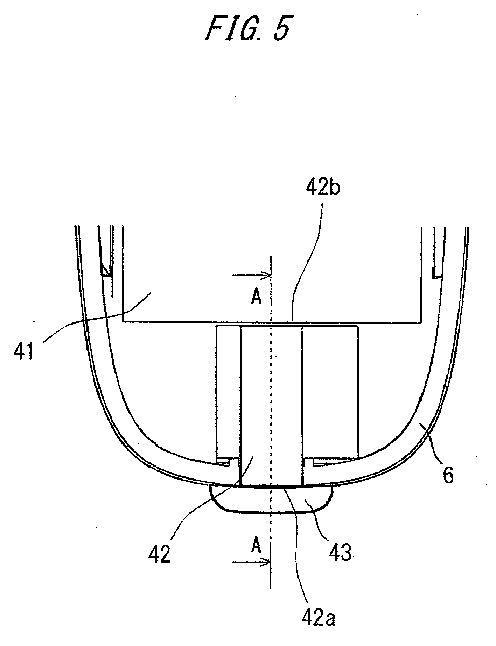

Specifically, Fig. 1A illustrates the configuration of the front side of the blood glucose level measurement device 1, and Fig. 1B the configuration of the rear side. A liquid crystal screen 4 for displaying measurement results and the like is provided on a panel 3 on the front side of the chassis 2. A test piece insertion groove 10 (corresponding to the fitting-removal section according to the present invention), into which there is inserted (as illustrated in Fig. 5 below) a test piece for measurement used in the blood glucose level measurement device 1, is provided at an end portion of the chassis 2. The test piece comprises a sensor that reacts with glucose in the blood sampled from the user, and that outputs an electric signal. To perform a blood glucose level measurement, the user places some blood of his/her own on the sensor, whereupon the measurement is carried out by the blood glucose level measurement device 1. As the sensor there can be used, for instance, a sensor that relies on glucose oxidase (GOD), as an oxidoreductase, and that employs a hydrogen peroxide electrode as an electrode for signal output. The configuration of the test piece itself is a conventional one and not a core feature of the present invention, and hence a detailed explanation thereof will be omitted in the present specification.

On the side face of the rear panel 3 of the chassis 2 there are provided, as illustrated in Fig. 1B, an operation button 7 for operating the blood glucose level measurement device 1, and a communication port B that connects, in a manner that enables communication, the blood glucose level measurement device 1 and an external device, for instance a computer or the like disposed outside the blood glucose level measurement device 1. The operation button 7 enables operations for all the processes that are performed in the blood glucose level measurement device 1, through a combination of various operations by the user on the operation button 7, for instance pressing, sliding, sustained pressing and the like. The operation button 7 corresponds to the operation unit according to the present invention.

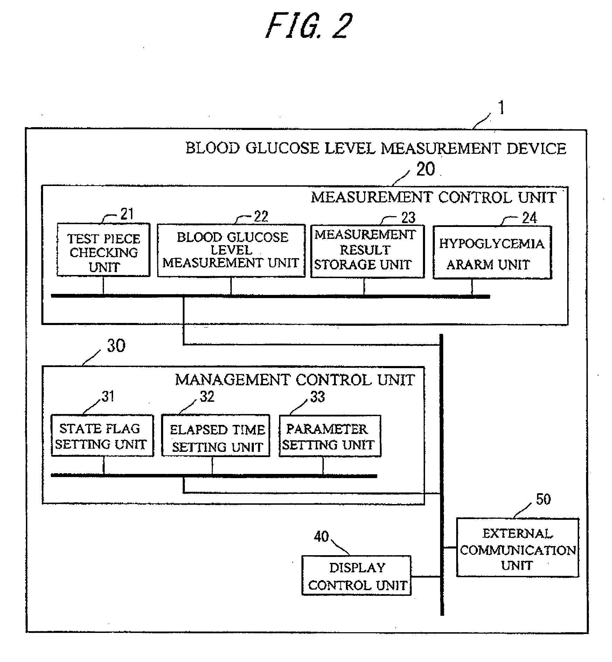

A control unit for controlling the various processes performed by the blood glucose level measurement device 1 is provided inside the latter. As the main control units of the blood glucose level measurement device 1 there are provided a measurement control unit 20, a management control unit 30, a display control unit 40 and an external communication unit 50, as depicted in Fig. 2. These control units are implemented by way of a computer program that is executed in a computer that is formed in the blood glucose level measurement device 1 and that comprises, for instance, a CPU, memory, a hard disk and the like, not shown. The above-described liquid crystal screen 4, operation button 7 and communication port 8 are appropriately connected to the control units illustrated in Fig. 2. As a result, there are executed various program processes relating to, for instance, display of measurement results to the user, transmission of operation instructions from the user to the measurement device, and outward communication of data stored in the blood glucose level measurement device 1. The features of the control units of the blood glucose level measurement device 1 of the present invention are explained further on.

As described above, the control units of the blood glucose level measurement device 1 are formed by the measurement control unit 20 that performs mainly measurement control relating to the measurement of blood glucose levels; the management control unit 30 that performs management control relating to measurement results; the display control unit 40 that performs display control of the measurement results; and the external communication unit 50 that controls communication with the exterior of the measurement device. However, the blood glucose level measurement device 1 may be configured so as to include control units other than the above. Specifically, the measurement control unit 20 has a test piece checking unit 21, a blood glucose level measurement unit 22, a measurement result storage unit 23 and a hypoglycemia alarm unit 24. The test piece checking unit 21 is a functional unit that checks whether the above-described test piece for blood glucose level measurement is set on the blood glucose level measurement device 1 in a state that enables measurement. The blood glucose level measurement unit 22 is a functional unit that measures a blood glucose level of the user on the basis of an output signal from the checked test piece. The measurement result storage unit 23 is a functional unit for saving and storing data on the results from the measurement performed by the blood glucose level measurement unit 22 in a storage device (memory) in the blood glucose level measurement device 1. The hypoglycemia alarm unit 24 is a functional unit that issues hypoglycemia alarm to the user when the measured results are lower than a standard range for an adequate blood glucose level.

The management control unit 30 has a state flag setting unit 31, an elapsed time setting unit 32, and a parameter setting unit 33. The state flag setting unit 31 has the function of setting flags relating to the state of the body, upon blood glucose level measurement, that exerts an influence on the blood glucose level of the user. Management of the blood glucose level of the user can be performed efficiently thanks to such a function. In the present example, state flags for post-exercise, pre-meal and post-meal, are employed as flags that indicate the state of the body of the user. To set these state flags, the user operates the operation button 7, and follows the contents displayed on the liquid crystal screen 4. Specifically, the user selects, through button operation, from among four state flags, namely a post-exercise flag, a pre-meal flag, a post-meal flag and no state flag. The state flag according to that selection is stored, in the blood glucose level measurement device 1, associated with a measured blood glucose level. These state flags relate to states of the body that are strongly linked to the blood glucose level of the user. Therefore, associating state flags to the measured blood glucose level is extremely useful for the management of blood glucose levels.

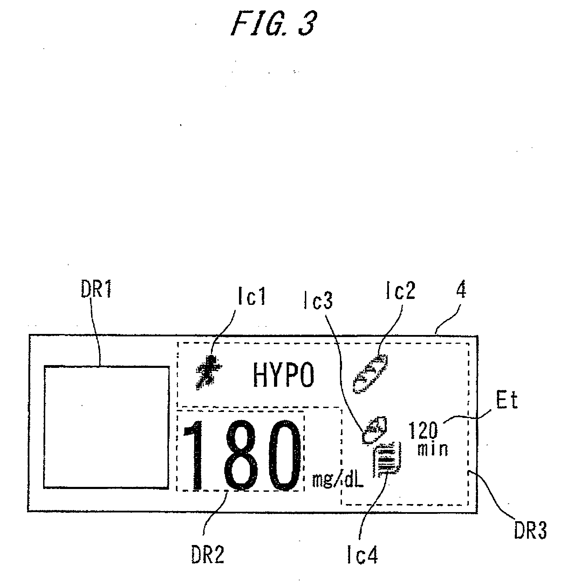

Display of these state flags on the liquid crystal screen 4 is accomplished by way of the display control unit 40. The display control unit 40 is a control unit for displaying, for instance, measurement results of blood glucose level by the measurement control unit 20, as well as state flags set by the management control unit 30, on a liquid crystal screen of the blood glucose level measurement device 1. Fig. 3 illustrates an example of display on the liquid crystal screen 4 as performed by the display control unit 40. To facilitate the explanation, the icons and numerical values are displayed collectively in the display screen example. A graph display region DR1, being a rectangular display region at which measurement results are displayed in the form of a graph, is formed at the left of the liquid crystal screen 4. At the center there is formed a measured numerical value display region DR2 at which measured blood glucose levels and the like are displayed. An icon display region DR3 at which there are displayed icons having various meanings, as well as a predetermined elapsed time, is formed around the measured numerical value display region DR2. The icons displayed at the icon display region DR3 include an icon "HYPO" for hypoglycemia alarm, as well as a post-exercise flag icon Ic1, a pre-meal flag icon Ic2, a post-meal flag first icon Ic3 and a second icon Ic4, as the above-described state flags. An elapsed time after meal Et is displayed, alongside the post-meal flag first and second icons, on the icon display region DR3. The display control itself of the display control unit 40 is not a core feature of the present invention, and hence a detailed explanation thereof will be omitted in the present specification.

In a case where the state flag set by the state flag setting unit 31 is a post-meal flag, the user can set information relating to the food intake amount ingested during a meal in the blood glucose level measurement device 1 according to the present example. Specifically, the user can subjectively select, as the food intake amount, one from among three benchmarks, namely 50%, 80% and 100%, where 100% denotes a full-stomach meal. In the blood glucose level measurement device 1 according to the present example, selection of this post-meal flag is displayed at the icon display region DR3 of Fig. 3 in the form of the post-meal flag first icon Ic3. Display of food intake amount is accomplished by modifying the number of bars that are displayed in the icon simultaneously with the display of the post-meal flag second icon Ic4. In a case, for instance, of 100% food intake amount, the number of bars is three (situation illustrated in Fig. 3), while for 80% and 50% of food intake amount the number of bars is two and one, respectively. By associating thus data relating to food intake amount with post-meal flags, the user can learn which meal amount corresponds to the measured blood glucose level. The user can grasp thus the relationship between food intake amount and blood glucose level, so that health management by the user relating to blood glucose levels is made easier in terms of, for instance, adjustment of food intake amount.

The elapsed time setting unit 32 is a functional unit that sets the elapsed time from the point in time at which a meal is over, upon setting of the post-meal state flag in the state flag by setting unit 31. Prior to a measurement, the elapsed time setting unit 32 sets an elapsed time counter that counts automatically the elapsed time since the point in time at which a meal is over, and associates the time elapsed, counted by the counter, of the blood glucose level of the measurement results at that point in time. The elapsed time is displayed as an elapsed time after meal Et in the icon display region DR3 of Fig. 3. Thereby, the user can check, in addition to the food intake amount, how long after the end of a meal the measured blood glucose level corresponds to. This feature further facilitates health management relating to blood glucose levels. The parameter setting unit 33 is a functional unit that sets various control parameters that are used by the blood glucose level measurement device 1. Examples of the parameters include, for instance, the time and date in the blood glucose level measurement device 1, as well as a container-unsealing date of a below-described test piece. The functional units in such a management control unit 30 allow the user to effectively manage, on his/her own, measurement results relating to blood glucose levels.

The external communication unit 50 is a control unit for transmission, in particular of measurement results by the measurement control unit 20, and information such as state flags managed by the management control unit 30, to the exterior of the blood glucose level measurement device 1. In the blood glucose level measurement device 1 of the present invention, communication control by the external communication unit 50 is not a core feature. Therefore, description is made only for the external communication function implemented by the external communication unit 50 in the present specification, and a detailed explanation thereof is omitted.

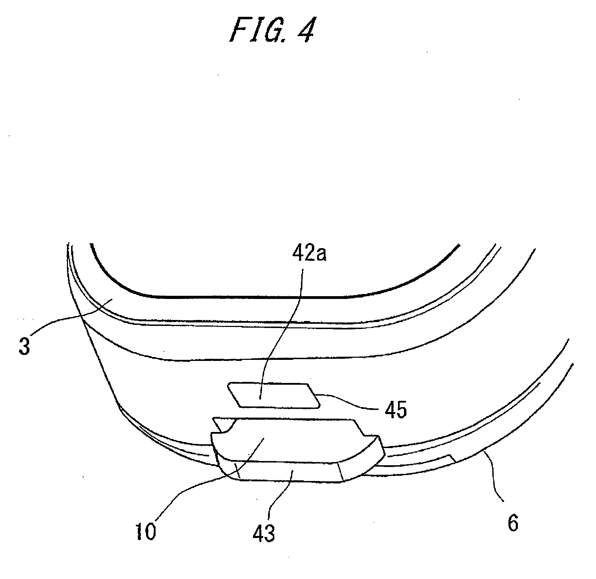

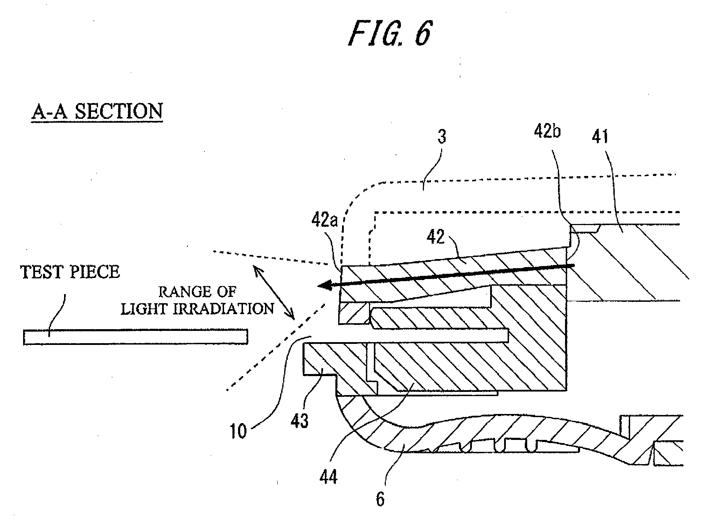

An explanation follows next, on the basis of Fig. 4 to Fig. 6, on the area of the test piece insertion groove 10 at which a test piece for blood glucose level measurement is inserted in the blood glucose level measurement device 1. Fig. 4 is an enlarged diagram of the outer appearance of the blood glucose level measurement device 1 in the vicinity of the test piece insertion groove 10. Fig. 5 is a top-view diagram of the blood glucose level measurement device 1 in a state where the front panel 3 is removed. Fig. 6 is a cross-sectional diagram along line A-A of Fig. 5 (the panel 3 is depicted as a broken line). A backlight 41 is provided at the rear face of the liquid crystal screen 4 of the blood glucose level measurement device 1. The backlight 41 has a plate-like shape, and emits light upon receiving power from a power source for blood glucose level measurement in the blood glucose level measurement device 1. The backlight 41 is an edge light-type backlight having a light source at an end portion side that is an opposite side to the test piece insertion groove 10, in the longitudinal direction of the blood glucose level measurement device 1. The backlight 41 corresponds to the light source section of the present invention.

An illumination opening 45 is provided above (i.e. towards the device front side at which the liquid crystal screen 4 is disposed) the test piece insertion groove 10 that is provided on the side face of the chassis 2 in the longitudinal direction of the blood glucose level measurement device 1. The light guide member 42 is provided in the blood glucose level measurement device 1 in such a manner that an end 42a of the light guide member 42 is exposed at the illumination opening 45. The light guide member 42 has a thin, long shape, and has end portions 42a, 42b into/out of which light can enter/exit. The light guide member 42 allows light to be incident into one end and to exit through the other end. The lateral portion of the light guide member 42 (portion other than the end portions and that extends in the longitudinal direction) is preferably subjected to a surface treatment such that light incident into the light guide member 42 does not leak readily through the lateral portion. The light guide member 42 thus formed is disposed adjacent to the backlight in such a manner that light emitted by the backlight 41 can strike the end 42b of the light guide member 42, as illustrated in Fig. 5 and Fig. 6. The light guide member 42 is also disposed in such a manner that the other end 42a is exposed out of the blood glucose level measurement device 1 at the illumination opening 45. Specifically, the light guide member 42 is disposed in the blood glucose level measurement device 1 in such a manner so as to connect the backlight 41 of the liquid crystal screen 4 disposed at the center of the front side panel 3, as illustrated in Fig. 1A, with the illumination opening 45 disposed on a side of the chassis 2.

As illustrated in Fig. 6, the light guide member 42 is disposed in the blood glucose level measurement device 1 so as to slant slightly, i.e. in such a manner that the end 42b is positioned slightly higher than the end 42a in Fig. 6. The direction of light that is guided within the light guide member 42 is defined thereby. In the present example, this slanting causes the light from the end 42a to be irradiated downwards in Fig. 6, i.e. towards the side at which the test piece is present. Thus the range of light irradiation from the end 42a can be adjusted to some extent through adjustment of the inclination of the light guide member 42.

A guide member 43 having a protrusion projecting slightly out of the device is disposed under the test piece insertion groove 10. The user can easily slip the test piece into the test piece insertion groove 10 thanks to the protrusion of the guide member 43. Once the test piece inserted into the test piece insertion groove 10 becomes electrically connected to the connection portion 44 provided at the back of the groove, there can take place the above-described checking of test piece insertion by the test piece checking unit 21, transmission of measurement results in the test piece to the device main body, and measurement of blood glucose level by the blood glucose level measurement unit 22.

In the blood glucose level measurement device 1 having the above-described configuration, light from the backlight 41, which is the light source for the liquid crystal screen 4, is transmitted by the light guide member 42 and is irradiated to the outside of the device through the illumination opening 45. The guide member 43 is positioned under the illumination opening 45, and hence the above illumination strikes the vicinity of the inlet portion of the test piece insertion groove 10. As a result, fitting of the test piece to the blood glucose level measurement device 1 becomes easier. In particular, an illumination method that utilizes light from the backlight 41, via the light guide member 42, is very useful upon measurement of blood glucose levels in dimly-lit rooms, for instance at nighttime. No dedicated illumination device for test piece insertion is provided in the blood glucose level measurement device 1 according to the present invention. Instead, light from the backlight of the liquid crystal screen 4 is effectively used to a greater degree, thanks to the light guide member 42. As a result, the configuration of the measurement device can be simplified, and consumption of current required for illumination can be reduced. This is preferable in terms of securing a power supply for driving the measurement device. In particular, the portable blood glucose level measurement device 1 combines the effects of securing driving power supply and enhancing user-friendliness. The desirability of the blood glucose level measurement device 1 in practice should therefore be appreciated. In the above-described examples, the backlight 41 and the light guide member 42 are configured separately, and are disposed adjacent to each other, but it is also possible, in an alternative configuration, to form the backlight 41 and the light guide member 42 continuously in the form of a single slab.

Herein, the backlight 41 emits light in response to an operation of the operation button 7 by the user. Specifically, the backlight 41 can be lighted up as a result of pushing, sliding or the like, of the operation button 7. Thereupon, light emitted by the backlight 41 is transmitted via the light guide member 42 and is irradiated through the illumination opening 45. That is, the operation button 7 is a button for transmission of instructions by the user for performing the various controls described above based on Fig. 2, and is also a button for triggering irradiation of light from the illumination opening 45.

The explanation of the above-described examples deals with illumination from the illumination opening 45 upon fitting of the test piece. However, the above-described illumination can take place, through operation of the operation button 7, also upon removal of the fitted test piece. Blood of the user may be placed on the test piece before fitting of the test piece or after fitting of the test piece. In the latter case, the test piece is fitted to the test piece insertion groove 10, and in that state, the user operates the operation button 7, whereupon the backlight 41 emits light that is irradiated through the illumination opening 45, to illuminate thereby the site at which the blood is placed on the test piece. The user can perform as a result the blood placement operation in an easy manner. In another method, the backlight 41 may be caused to emit light automatically at the same time that the test piece is inserted into the test piece insertion groove 10, or at a timing after a predetermined time has elapsed after fitting of the test piece, irrespective of the presence or absence of illumination during placement on the test piece.

In terms of controlling power consumption, it is not desirable for the backlight 41 to be lighted up at all times. For instance, power consumption in the backlight 41 can be curbed by controlling the backlight 41 in such a manner that the latter lights up after a predetermined time has elapsed since some operation of the operation button 7 by the user. In order to effectively support the operations of sensor fitting and removal by the user, the luminous intensity at the illumination opening 45 ranges preferably from 1 µCd/m² to 10000 Cd/m². more preferably from 1 mCd/m² to 2000 Cd/m², and yet more preferably from 1 Cd/m² to 100 Cd/m².

In the above-described example light from the backlight 41, which is the light source for the liquid crystal screen 4, is used, by way of the light guide member 42, as a light source for test piece insertion. However, if a light source other than the backlight 41 is present in the blood glucose level measurement device 1, light emitted by that other light source may be guided to the illumination opening 45 by way of a light guide member. Such a configuration falls also within the scope of the invention.

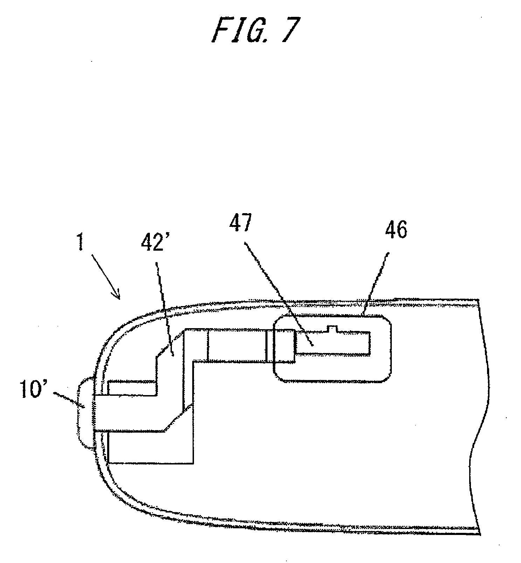

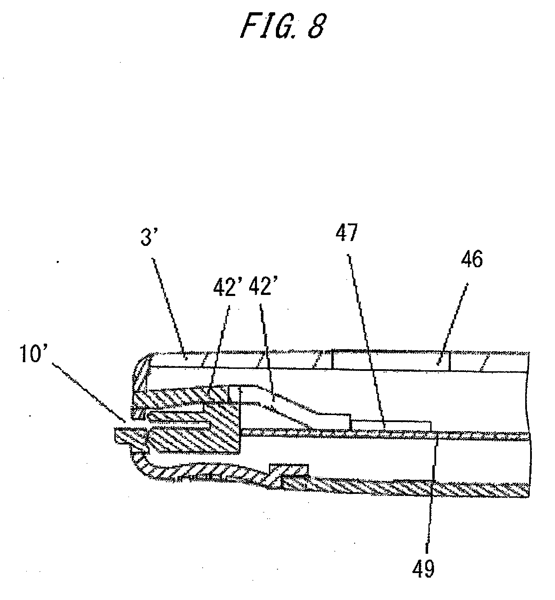

A blood glucose level measurement device 1 according to a variation of the present example will be explained next. Fig. 7 and Fig. 8 are cross-sectional diagrams illustrating relevant portions of a measurement device 1 according to the present variation. Fig. 7 is a diagram illustrating schematically a horizontal cross section (transversal cross section) of the blood glucose level measurement device 1. Fig. 8 is a diagram illustrating schematically a vertical cross section (longitudinal cross section) of the measurement device 1. The blood glucose level measurement device 1 according to the present variation comprises an operation unit 46 for operating the blood glucose level measurement device 1, a light source 47 for light-up of the operation unit 46 , and a light guide member 42' for guiding light from the light source 47 to a test piece insertion groove 10'. Fig. 7 illustrates the plan-view arrangement relationship between the operation unit 46, the light source 47 and the light guide member 42' of the blood glucose level measurement device 1. Fig. 8 illustrates a perpendicular cross section of Fig. 7.

As illustrated in the figures, the operation unit 46 is provided in a panel 3' that makes up the front face of a chassis of the blood glucose level measurement device 1. The light source 47 for irradiating the operation unit 46 is disposed on a base 49 in the blood glucose level measurement device 1, below the operation unit 46. As illustrated in the figures, the light guide member 42' is formed between the light source 47 and the test piece insertion groove 10', in such a manner that light from the light source 47 is guided to the test piece insertion groove 10'. In this example, the operation unit 46 is formed of a material that is brightly defined by the light emitted by the light source 47. The operation unit 46 is lighted-up through light emission by the light source 47, and at the same time, the light from the light source 47 is guided to the test piece insertion groove 10' by way of the light guide member 92'. In yet another variation, the blood glucose level measurement device 1 may have a light source for irradiation of the above-described communication port 8, and there is provided a light guide member that guides light from this light source to the test piece insertion groove. So long as light from a light source is guided by way of a light guide member, such a configuration is found to lie within the scope of the present invention, even if the light source is not a light source provided for test piece insertion, as in the above-described variations. An exposed end portion of the light guide member may be arranged arbitrarily so as to irradiate light that is guided from the light source, by way of the light guide member, onto a position in the vicinity of the operation button 7 and/or the communication port 8. The operation of the operation button 7 by the user, as well as access to the communication port 8, can be assisted thereby.

## Claims

1. A biological information measurement device (1) for measuring biological information of a user, comprising:
a device main body (2);
a fitting-removal section (10) which is provided in the device main body (2), and to/from which a sensor that measures the biological information can be fitted/removed;
a light source section (41, 47) that is provided within the device main body (2) at a position spaced apart from the fitting-removal section (10) and that functions as a light source for components of the biological information measurement device (1) other than the fitting-removal section (10) **characterised in that** the device further comprises:
an elongate light guide member (42, 42') having a first end portion (42b) in a vicinity of the light source section (41, 47) and a second end portion (42a) in a vicinity of the fitting-removal section (10), the elongate light guide member (42, 42') extending from the first end portion (42b) to the second end portion (42a) and configured to guide light emitted by the light source section (41, 47) to a predetermined position in the vicinity of the fitting-removal section (10) to/from which the sensor can be fitted/removed, wherein the elongate light guide member (42, 42') is arranged such that, in use, light emitted by the light source section (41, 47) is incident into the first end portion (42b), is guided along the elongate light guide member (42, 42'), and exits through the second end portion (42a).

2. The biological information measurement device (1) according to claim 1, further comprising:
a display (4) configured to display biological information measured by way of the sensor,
wherein the light source section (41, 47) is a backlight (41) that functions as a light source for the display (4).

3. The biological information measurement device (1) according to claim 2,
wherein the fitting-removal section (10) is provided on a side face of the device main body (2), and
the light guide member (42, 42') has a length enabling light from the backlight to be guided to the predetermined position.

4. The biological information measurement device (1) according to any one of claims 1 to 3, further comprising:
an operation unit (46) configured to enable a user to operate the biological information measurement device (1),
wherein upon operation of the operation unit (46) by the user, the light source section (41, 47) emits light and the light guide member (42, 42') guides the light to the predetermined position.

5. The biological information measurement device (1) according to claim 4,
wherein upon fitting of the sensor to the fitting-removal section (10), the light source section (41, 47) emits light and the light guide member (42, 42') guides the light to the predetermined position.

6. The biological information measurement device (1) according to any one of claims 1 to 5,
wherein the biological information measurement device (1) is a device configured to measure a blood glucose level of a user, and
a sensor configured for blood glucose level measurement, on which blood of the user can be placed and which produces a current output according to a glucose level of the blood, can be fitted to/removed from the fitting-removal section (10).

7. The biological information measurement device (1) according to any one of claims 1 to 6,
wherein the biological information measurement device (1) is a user-portable device.

8. An illumination method in a biological information measurement device (1) for measuring biological information of a user and that has a device main body (2) and a fitting-removal section (10) to/from which a sensor that measures biological information of a user is fitted/removed from outside, the illumination method being a method for illuminating a vicinity of the fitting-removal section (10) and comprising:
a first step of causing light emission by a light source section (41, 47) that is provided within the device main body (2) at a position spaced apart from the fitting-removal section (10) and that functions as a light source for components of the biological information measurement device (1) other than the fitting-removal section (10) ; and **characterised in that**
it comprises a second step of guiding light emitted by the light source section (41, 47) in the first step, to a predetermined position in the vicinity of the fitting-removal section (10) to/from which the sensor is fitted/removed, by way of an elongate light guide member (42, 42') having a first end portion (42b) in a vicinity of the light source section (41, 47) and a second end portion (42a) in the vicinity of the fitting-removal section (10), the elongate light guide member (42, 42') extending from the first end portion (42b) to the second end portion (42a), wherein the light emitted by the light source section (41, 47) is incident into the first end portion (42b), is guided along the elongate light guide member (42, 42'), and exits through the second end portion (42a).

## Patentansprüche

1. Messvorrichtung für biologische Informationen (1) zum Messen biologischer Informationen eines Benutzers, wobei die Vorrichtung Folgendes umfasst:
einen Vorrichtungshauptkörper (2);
einen Anbringungs- und Entfernungsabschnitt (10), der in dem Vorrichtungshauptkörper (2) vorgesehen ist, und an dem ein Sensor angebracht bzw. von dem ein Sensor entfernt werden kann, der die biologischen Informationen misst;
einen Lichtquellenabschnitt (41, 47), der innerhalb des Vorrichtungshauptkörpers (2) an einer Position vorgesehen ist, die von dem Anbringungs- und Entfernungsabschnitt (10) beabstandet angeordnet ist und als Lichtquelle für die Komponenten der Messvorrichtung für biologische Informationen (1) dient, die sich von dem Anbringungs- und Entfernungsabschnitt (10) unterscheiden; **dadurch gekennzeichnet, dass** die Vorrichtung ferner Folgendes umfasst:
ein längliches Lichtleiterelement (42, 42'), dessen erster Endabschnitt (42b) in der Nähe des Lichtquellenabschnitts (41, 47) angeordnet ist und dessen zweiter Endabschnitt (42a) in der Nähe des Anbringungs- und Entfernungsabschnitts (10) angeordnet ist, wobei sich das längliche Lichtleiterelement (42, 42') von dem ersten Endabschnitt (42b) zu dem zweiten Endabschnitt (42a) erstreckt und so konfiguriert ist, dass es Licht, das von dem Lichtquellenabschnitt (41, 47) abgegeben wird, zu einer vorbestimmten Position in der Nähe des Anbringungs- und Entfernungsabschnitts (10) zu leiten, an dem der Sensor angebracht bzw. von dem der Sensor entfernt werden kann, wobei das längliche Lichtleiterelement (42, 42') so angeordnet ist, dass es bei Gebrauch Licht, das von dem Lichtquellenabschnitt (41, 47) abgegeben wird, auf den ersten Endabschnitt (42b) auftrifft und entlang des länglichen Lichtleiterelements (42, 42') geleitet wird und durch den zweiten Endabschnitt (42a) austritt.

2. Messvorrichtung für biologische Informationen (1) nach Anspruch 1, die ferner Folgendes umfasst:
eine Anzeige (4), die so konfiguriert ist, dass sie biologische Informationen anzeigt, die mit Hilfe des Sensors gemessen werden,
wobei der Lichtquellenabschnitt (41, 47) eine Hintergrundbeleuchtung (41) ist, die als Lichtquelle für die Anzeige (4) dient.

3. Messvorrichtung für biologische Informationen (1) nach Anspruch 2:
wobei der Anbringungs- und Entfernungsabschnitt (10) auf einer Seitenfläche des Vorrichtungshauptkörpers (2) vorgesehen ist, und
das Lichtleiterelement (42, 42') eine Länge aufweist, die ermöglicht, dass Licht von der Hintergrundbeleuchtung zu der vorbestimmten Position geleitet werden kann.

4. Messvorrichtung für biologische Informationen (1) nach einem der Ansprüche 1 bis 3, die ferner Folgendes umfasst:
eine Betätigungseinheit (46), die dafür konfiguriert ist, einem Benutzer zu ermöglichen, die Messvorrichtung für biologische Informationen (1) zu betätigen,
wobei der Lichtquellenabschnitt (41, 47) bei Betätigung der Betätigungseinheit (46) durch den Benutzer Licht abgibt und das Lichtleiterelement (42, 42') das Licht zu der vorbestimmten Position leitet.

5. Messvorrichtung für biologische Informationen (1) nach Anspruch 4:
wobei beim Anbringen des Sensors an dem Anbringungs- und Entfernungsabschnitt (10), der Lichtquellenabschnitt (41, 47) Licht abgibt und das Lichtleiterelement (42, 42') das Licht zu der vorbestimmten Position leitet.

6. Messvorrichtung für biologische Informationen (1) nach einem der Ansprüche 1 bis 5,
wobei die Messvorrichtung für biologische Informationen (1) eine Vorrichtung ist, die dafür konfiguriert ist, einen Blutzuckerspiegel des Benutzers zu messen, und
wobei ein Sensor, der für die Messung des Blutzuckerspiegels konfiguriert ist, auf dem Blut des Benutzers angeordnet werden kann und der einen Ausgangsstrom entsprechend einem Blutzuckerspiegel erzeugt, an dem Anbringungs- und Entfernungsabschnitt (10) angebracht und entfernt werden kann.

7. Messvorrichtung für biologische Informationen (1) nach einem der Ansprüche 1 bis 6,
wobei die Messvorrichtung für biologische Informationen (1) eine für den Benutzer tragbare Vorrichtung ist.

8. Beleuchtungsverfahren in einer Messvorrichtung für biologische Informationen (1) zum Messen biologischer Informationen eines Benutzers, die einen Vorrichtungshauptkörper (2) und einen Anbringungs- und Entfernungsabschnitt (10) aufweist, an dem von der Außenseite ein Sensor, der biologische Informationen eines Benutzers misst, angebracht und von dem ein Sensor entfernt wird, wobei das Beleuchtungsverfahren ein Verfahren zum Beleuchten einer Umgebung des Anbringungs- und Entfernungsabschnitts (10) ist, und wobei das Verfahren Folgendes umfasst:
einen ersten Schritt zum Veranlassen der Lichtemission von einem Lichtquellenabschnitt (41, 47), der in dem Vorrichtungshauptkörper (2) an einer Position vorgesehen ist, die von dem Anbringungs- und Entfernungsabschnitt (10) beabstandet ist und als Lichtquelle für die Komponenten der Messvorrichtung für biologische Informationen (1) dient, die sich von dem Anbringungs- und Entfernungsabschnitt (10) unterscheiden; und **dadurch gekennzeichnet, dass** es Folgendes umfasst:
einen zweiten Schritt zum Leiten von Licht, das von dem Lichtquellenabschnitt (41, 47) in dem ersten Schritt abgegeben wird, zu einer vorbestimmten Position in der Nähe des Anbringungs- und Entfernungsabschnitts (10), an dem der Sensor angebracht und von dem der Sensor entfernt wird, und zwar mit Hilfe eines länglichen Lichtleiters (42, 42'), dessen erster Endabschnitt (42b) in der Nähe des Lichtquellenabschnitts (41, 47) angeordnet ist und dessen zweiter Endabschnitt (42a) in der Nähe des Anbringungs- und Entfernungsabschnitts (10) angeordnet ist, wobei sich das längliche Lichtleiterelement (42, 42') von dem ersten Endabschnitt (42b) zu dem zweiten Endabschnitt (42a) erstreckt, wobei das Licht, das von dem Lichtquellenabschnitt (41, 47) abgegeben wird, auf den ersten Endabschnitt (42b) auftrifft und entlang des länglichen Lichtleiterelements (42, 42') geleitet wird und durch den zweiten Endabschnitt (42a) austritt.

## Revendications

1. Dispositif de mesure d'informations biologiques (1) permettant de mesurer des informations biologiques d'un utilisateur, comportant :
un corps principal de dispositif (2) ;
une section de mise en place et de retrait (10) qui est mise en oeuvre dans le corps principal de dispositif (2), et sur laquelle/en provenance de laquelle un capteur qui mesure les informations biologiques peut être mis en place/retiré ;
une section de source de lumière (41, 47) qui est mise en oeuvre à l'intérieur du corps principal de dispositif (2) au niveau d'une position espacée par rapport à la section de mise en place et de retrait (10) et qui tient lieu de source de lumière pour des composants du dispositif de mesure d'informations biologiques (1) autres que la section de mise en place et de retrait (10) ;
**caractérisé en ce que** le dispositif comporte par ailleurs :
un élément de guidage de lumière allongé (42, 42') ayant une première partie d'extrémité (42b) dans une région à proximité de la section de source de lumière (41, 47) et une seconde partie d'extrémité (42a) dans une région à proximité de la section de mise en place et de retrait (10), l'élément de guidage de lumière allongé (42, 42') s'étendant depuis la première partie d'extrémité (42b) jusqu'à la seconde partie d'extrémité (42a) et étant configuré à des fins de guidage de la lumière émise par la section de source de lumière (41, 47) jusqu'à une position prédéterminée dans une région à proximité de la section de mise en place et de retrait (10) sur laquelle/en provenance de laquelle le capteur peut être mis en place/retiré, dans lequel l'élément de guidage de lumière allongé (42, 42') est agencé de telle sorte que, lors de l'utilisation, la lumière émise par la section de source de lumière (41, 47) est incidente dans la première partie d'extrémité (42b), est guidée le long de l'élément de guidage de lumière allongé (42, 42'), et sort par la seconde partie d'extrémité (42a).

2. Dispositif de mesure d'informations biologiques (1) selon la revendication 1, comportant par ailleurs :
un affichage (4) configuré à des fins d'affichage des informations biologiques mesurées par le biais du capteur,
dans lequel la section de source de lumière (41, 47) est un dispositif de rétroéclairage (41) qui tient lieu de source de lumière pour l'affichage (4).

3. Dispositif de mesure d'informations biologiques (1) selon la revendication 2,
dans lequel la section de mise en place et de retrait (10) est mise en oeuvre sur une face latérale du corps principal de dispositif (2), et
l'élément de guidage de lumière (42, 42') a une longueur permettant à la lumière du dispositif de rétroéclairage d'être guidée jusqu'à la position prédéterminée.

4. Dispositif de mesure d'informations biologiques (1) selon l'une quelconque des revendications 1 à 3, comportant par ailleurs :
une unité de fonctionnement (46) configurée pour permettre à un utilisateur de faire fonctionner le dispositif de mesure d'informations biologiques (1),
dans lequel, lors du fonctionnement de l'unité de fonctionnement (46) par l'utilisateur, la section de source de lumière (41, 47) émet de la lumière et l'élément de guidage de lumière (42, 42') guide la lumière jusqu'à la position prédéterminée.

5. Dispositif de mesure d'informations biologiques (1) selon la revendication 4,
dans lequel, lors de la mise en place du capteur sur la section de mise en place et de retrait (10), la section de source de lumière (41, 47) émet de la lumière et l'élément de guidage de lumière (42, 42') guide la lumière jusqu'à la position prédéterminée.

6. Dispositif de mesure d'informations biologiques (1) selon l'une quelconque des revendications 1 à 5,
dans lequel le dispositif de mesure d'informations biologiques (1) est un dispositif configuré à des fins de mesure d'un niveau de glucose sanguin d'un utilisateur, et
un capteur configuré à des fins de mesure du niveau de glucose sanguin, sur lequel du sang de l'utilisateur peut être placé et qui produit une sortie de courant en fonction du niveau de glucose dans le sang, peut être mis en place sur/retiré en provenance de la section de mise en place et de retrait (10) .

7. Dispositif de mesure d'informations biologiques (1) selon l'une quelconque des revendications 1 à 6,
dans lequel le dispositif de mesure d'informations biologiques (1) est un dispositif portable par l'utilisateur.

8. Procédé d'éclairage dans un dispositif de mesure d'informations biologiques (1) permettant de mesurer des informations biologiques d'un utilisateur et qui a un corps principal de dispositif (2) et une section de mise en place et de retrait (10) sur laquelle/en provenance de laquelle un capteur qui mesure des informations biologiques d'un utilisateur peut être mis en place/retiré depuis l'extérieur, le procédé d'éclairage étant un procédé permettant d'éclairer une région à proximité de la section de mise en place et de retrait (10) et comportant :
une première étape consistant à entraîner une émission de lumière par une section de source de lumière (41, 47) qui est mise en oeuvre à l'intérieur du corps principal de dispositif (2) au niveau d'une position espacée par rapport à la section de mise en place et de retrait (10) et qui tient lieu de source de lumière pour des composants du dispositif de mesure d'informations biologiques (1) autres que la section de mise en place et de retrait (10) ; et **caractérisé en ce qu'**il comporte une seconde étape consistant à guider la lumière émise par la section de source de lumière (41, 47) au cours de la première étape, jusqu'à une position prédéterminée dans une région à proximité de la section de mise en place et de retrait (10) sur laquelle/en provenance de laquelle le capteur peut être mis en place/retiré, par le biais d'un élément de guidage de lumière allongé (42, 42') ayant une première partie d'extrémité (42b) dans une région à proximité de la section de source de lumière (41, 47) et une seconde partie d'extrémité (42a) dans une région à proximité de la section de mise en place et de retrait (10), l'élément de guidage de lumière allongé (42, 42') s'étendant depuis la première partie d'extrémité (42b) jusqu'à la seconde partie d'extrémité (42a), dans lequel la lumière émise par la section de source de lumière (41, 47) est incidente dans la première partie d'extrémité (42b), est guidée le long de l'élément de guidage de lumière allongé (42, 42'), et sort par la seconde partie d'extrémité (42a).
